# EUROPÄISCHE PATENTSCHRIFT

(11) **EP 0 417 579 B1**
(45) Veröffentlichungstag und Bekanntmachung des Hinweises auf die Patenterteilung: **03.11.1993**
(21) Anmeldenummer: 90116804.7
(22) Anmeldetag: 01.09.1990
(51) Int. Cl.: A61M 1/14

(54) **Druckausgleichgefäss für ein Hämodialysekonzentrat**
Surge tank for a haemodialysis concentrate
Réservoir d'égalisation de pression pour concentré d'hémodialyse

(30) Priorität: 09.09.1989 DE 3930181
(43) Veröffentlichungstag der Anmeldung: 20.03.1991
(73) Patentinhaber: Fresenius AG, 61350 Bad Homburg (DE)
(72) Erfinder: Plaschegg, Hans-Dietrich, Dr., D-6370 Oberursel (DE); Backhaus, Wendelin, Dipl.-Phys., D-6242 Kronberg 2 (DE); Manke, Joachim, Dipl.-Phys., D-6293 Löhnberg 2 (DE); Pieper, Walter, Dipl.-Ing., D-6364 Florstadt 1 (DE); Walter, Hans, D-6050 Offenbach (DE)
(74) Vertreter: Fuchs Mehler Weiss

(56) Entgegenhaltungen:
- DE-A- 3 215 003
- DE-A- 3 307 112
- DE-A- 3 443 911
- DE-A- 3 740 598
- DE-B- 1 118 480
- DE-C- 3 734 880

## Beschreibung

Die Erfindung betrifft ein Druckausgleichsgefäß gemäß dem Oberbegriff des Anspruchs 1.

Bei der Hämodialyse wird die Dialysierflüssigkeit üblicherweise durch Mischung von Dialysekonzentrat mit Wasser hergestellt.

Bei der Herstellung oder Aufbereitung von Dialysierflüssigkeit werden zwei Verfahren unterschieden:

Bei dem ersten Verfahren erfolgt die Mischung mittels eines volumetrischen Mischverfahrens, bei welchem Wasser und Konzentrat in einem bestimmten vorgegebenen volumetrischen Verhältnis miteinander gemischt werden, beispielsweise in einem Verhältnis von 1:34.

Ein zweites Verfahren zur Herstellung von Dialysierflüssigkeit ist das leitfähigkeitsgeregelte Verfahren, bei welchem das Verhältnis des Wasser- und Konzentratstroms so geregelt wird, daß sich in der fertigen Dialysierflüssigkeit eine gewisse Leitfähigkeit einstellt.

Üblicherweise wird die Dialysierflüssigkeit aus einem oder zwei Konzentraten und Wasser zubereitet, es ist jedoch auch möglich, die Dialysierflüssigkeit aus mehr als zwei Konzentraten und Wasser zuzubereiten.

In der Praxis erfolgt die Bereitstellung des Dialysekonzentrats auf zwei unterschiedliche Arten. Zum einen ist es möglich, das Dialysekonzentrat, wie üblich, in Kanistern bereitzustellen und unter Verwendung einer Konzentratpumpe des Hämodialysegeräts anzusaugen und einer Mischeinrichtung zuzuführen in welcher das Dialysekonzentrat mit aufbereitetem Wasser gemischt wird. Diese Form der Aufbereitung der Dialysierflüssigkeit gestattet eine gewisse Flexibilität bei der Auswahl der Zusammensetzung der Dialysierflüssigkeit, bedingt andererseits jedoch einen erheblichen Arbeitsaufwand, um die Kanister zu transportieren, bereitzustellen und zu entsorgen. Weiterhin ist es jeweils erforderlich, die Kanister in geeigneter Weise mit einer Konzentratansaugleitung zu verbinden. Auch hieraus können sich Fehlerquellen ergeben. Ein beträchtliches Problem bereitet die Entsorgung der leeren Kanister, zum einen wegen der dabei auftretenden Abfallmenge als solcher und zum anderen wegen des relativ großen Volumens der leeren Kanister.

Es wurden deshalb zentrale Konzentratversorgungen entwikkelt, bei welchen die Konzentrate von einem zentralen Behälter unter Druck zu jeweiligen Zapfstellen oder Anschlußstellen der Hämodialysegeräte gefördert werden.

Die üblicherweise bei Hämodialysegeräten verwendeten Konzentratpumpen sind so aufgebaut, daß deren Förderrate vom Eingangsdruck beeinflußt wird. Es ist deshalb erforderlich, den üblicherweise schwankenden Druck einer zentralen Konzentratversorgung zu vergleichmäßigen, um die Föderrate der Mischpumpe nicht durch Druckschwankungen zu beeinträchtigen. Es wurden deshalb Druckausgleichsbehälter geschaffen, welche aus einem belüftbaren Gefäß sowie einem Niveauregelsystem bestehen. So werden beispielsweise Schwimmerschalter verwendet, um ein Konzentrateinlaßventil und/oder ein Ventil in einer Auslaßleitung zum Hämodialysegerät zu betätigen.

Es sind aus dem Stand der Technik Druckausgleichsbehälter bekannt, welche seitlich am Ausgleichsgefäß oder im Boden desselben einen Anschluß für eine Konzentratansaugleitung aufweisen. Die Konzentratansaugleitung kann jedoch auch in Form eines Ansaugrohres ausgebildet sein, welches in einen Konzentratkanister eingeführt werden kann, sowie dies aus der DE-A1-37 34 880 bekannt ist.

Die DE-A1 beschreibt eine Vorrichtung für die Hämodialyse, bei welcher in einer Mischeinrichtung Konzentrat aus zwei Behältern mit Wasser gemischt wird. Die Behälter sind in beliebiger Form ausgebildet, das Konzentrat wird jeweils durch ein Ansaugrohr abgepumpt. Wie bereits erwähnt, stellt die gezeigte Zuführung des Konzentrates und dessen Abpumpung aus einem Konzentratkanister ein nicht für alle Anwendungsfälle geeignetes Prinzip dar. Insbesondere bei einer kontinuierlichen Zuführung von Konzentrat aus einem größeren Tank kann sich, bedingt durch die Ausgestaltung der Pumpen und der Leitungen die beschriebene Entlüftungsmöglichkeit als nicht ausreichend erweisen.

Der Erfindung liegt die Aufgabe zugrunde, ein Druckausgleichsgefäß der eingangs genannten Art zu schaffen, welches bei einfachem Aufbau und sicherer Wirkungsweise einen Druckausgleich in der Zuführung des Hämodialysekonzentrats gewährleistet und welches den Anschluß des Hämodialysegerätes an eine zentrale Konzentratversorgung ermöglicht.

Die Aufgabe wird erfindungsgemäß durch die kennzeichnenden Merkmale des Anspruchs 1 gelöst.

Weitere vorteilhafte Ausgestaltungen der Erfindung ergeben sich aus den Unteransprüchen.

Das erfindungsgemäße Druckausgleichsgefäß zeichnet sich durch eine Reihe erheblicher Vorteile aus, so ist es erfindungsgemäß möglich, sowohl einen Druckausgleich vorzunehmen, als gleichzeitig ein bestimmtes Niveau des Hämodialysekonzentrats sicher zu stellen. Erfindungsgemäß ist es insbesondere möglich, die im Konzentratansaugrohr enthaltene bzw. diesem zugeordnete Niveaumeßeinrichtung zur Steuerung des Niveaus in dem Druckausgleichsgefäß zu verwenden.

Das erfindungsgemäße Druckausgleichsgefäß umfaßt ein im wesentlichen zylindrisches, aufrecht angeordnetes Gefäß, welches der Länge des Konzentratansaugerohrs angepaßt ist. Das zylindrische Rohr ist unten verschlossen und weist seitlich einen Einlaß für eine Konzentratzuführung auf. Der obere Bereich des zylindrischen Gefäßes ist offen, so daß das Konzentratansaugrohr eingesteckt werden kann, wobei dieses in gestecktem Zustand die Öffnung verdeckt, so daß keine Fremdkörper in das zylindrische Gefäß eindringen können. Für den Fall, daß das Konzentratansaugrohr entnommen wurde, kann das zylindrische Gefäß durch einen Deckel verschlossen werden. Weiterhin ist im oberen Bereich des zylindrischen Gefäßes eine Entlüftungsöffnung vorgesehen, welche vorteilhafterweise mittels einer hydrophoben Membran verschlossen ist.

Die Einlaßleitung im Bodenbereich des zylindrischen Gefäßes ist bevorzugterweise mittels eines Ventils zu öffnen bzw. zu verschließen. Mittels der Steuereinrichtung des Hämodialysegerätes ist es möglich, das Ventil aufgrund eines Signals des im Ansaugrohr intergrierten Niveausensors so zu steuern, daß das Konzentratflüssigkeitsniveau annähernd konstant bleibt und möglichst gering gehalten wird.

Sofern bei dem erfindungsgemäßen Druckausgleichsgefäß nur ein Niveausensor verwendet wird, welcher eine einzige Schaltschwelle besitzt, pendelt das Flüssigkeitsniveau um diese Schaltschwelle.

Bevorzugterweise ist erfindungsgemäß vorgesehen, daß eine Alarmeinrichtung betätigt wird, sofern nach Öffnen des Konzentrateingangsventils die Schaltschwelle des Niveausensors nicht innerhalb einer bestimmten Zeit überschritten wird. Dabei wird das Ventil geschlossen, um den Benutzer auf eine Störung hinzuweisen.

Bei einer derartigen Störung kann es sich entweder um eine Unterbrechung des Konzentratflusses handeln, bei welcher das Druckausgleichgefäß nicht mehr ausreichend befüllt wird, es ist jedoch auch möglich, daß der Niveausensor einen Defekt aufweist. Der letztere Fall würde dazu führen, daß durch das Konzentrat die hydrophobe Membran verstopft würde, so daß keine ausreichende Entlüftung stattfinden könnte.

Hinsichtlich des Niveausensors, welcher im Ansaugrohr integriert ist, ist es möglich, diesen so auszubilden, wie dies in der DE-OS 37 34 880 beschrieben ist.

Erfindungsgemäß ist es möglich, den Niveausensor, wie bekannt, in Form eines Leitfähigkeitsensor auszubilden. Der Niveausensor kann jedoch auch als faseroptischer Sensor ausgestaltet sein, um beispielsweise die optische Drehung des Konzentrats oder das Niveau in bekannter Weise über Totalreflexierung zu detektieren bzw. zu messen. Es ist jedoch auch möglich, den Niveausensor als Dichtesensor oder als Vibrationssensor auszubilden.

In einer besonders günstigen Ausgestaltung der Erfindung ist vorgesehen, daß das zylindrische Gefäß in Form eines im Hämodialysegerät integrierten Gefäßes ausgebildet ist, mittels dessen, das Ansaugrohr während der Desinfektions- und Reinigungsphase desinfizierbar und reinigbar ist. Diese Ausgestaltung weist den entscheidenden Vorteil auf, daß kein zusätzliches Gefäß verwendet werden muß, da das bereits bekannte Desinfektions- und Reinigungsgefäß nach entsprechender Umgestaltung zugleich als Druckausgleichsgefäß verwendet werden kann.

Im letzten Fall ist es erforderlich zwei weitere Anschlußleitungen an dem zylindrischen Gefäß vozusehen, welche über Ventile geöffnet bzw. geschlossen werden können. Die Zuleitung bzw. Ableitung dient zur Zuführung bzw. Abführung von Reinigungsflüssigkeit.

In letztgenannten Ausgestaltungsfall ist es besonders günstig, wenn das Druckausgleichgefäß so im Dialysierflüssigkeitskreislauf angeschlossen ist, daß es stromab des Dialysators noch vor einem eventuellen Rezirkulationskreislauf angeordnet ist.

Die Zuleitung bzw. Ableitung ist so angeordnet, daß das zylindrische Gefäß von der Reinigungsflüssigkeit voll oder im Nebenstrom durchspült werden kann und gleichzeitig die Reinigungsflüssigkeit auch von der Konzentratpumpe angesaugt werden kann. Während der Desinfektions- und Reinigungsphase bleibt das Ventil in der Konzentrationsflüssigkeitszuleitung geschlossen, weiterhin wird die Niveaumeßschaltung ausgeschaltet. Während der Dialyse sind die beiden Ventile in der Zuleitung bzw. Ableitung der Reinigungsflüssigkeit geschlossen, während das Ventil in der Konzentratszuleitung geöffnet ist.

In einer weiteren, besonders günstigen Ausgestaltung der Erfindung ist vorgesehen, daß an dem Druckausgleichsgefäß eine Sensoreinrichtung angeordnet ist, mittels derer festgestellt werden kann, ob das Konzentratansaugrohr in korrekter Weise in das zylindrische Gefäß eingesteckt bzw. an diesem fixiert ist. Die Sensoreinrichtung kann beispielsweise einen Magnetschalter umfassen, welcher durch einen an dem Konzentratansaugrohr befestigten Magnet betätigt wird. Dabei kann der Magnetschalter bzw. die Sensoreinrichtung so mit der Steuereinrichtung verbunden sein, daß das Konzentrateinlaßventil nur dann angesteuert werden kann, wenn das Konzentratansaugrohr in korrekter Weise angeordnet ist.

Bei dem letztbeschriebenen Ausführungsbeispiel, bei welchem das zylindrische Gefäß zugleich zur Desinfektion oder Reinigung dienen kann, ist es möglich, die zentrale Konzentratversorgung stets in einem angeschlossenen Zustand zu belassen. Es ist ohne mechanische Betätigung, das heißt ohne Umstecken von Rohrverbindungen oder ähnlichem möglich, eine Dialyse durchzuführen und nachfolgend das Dialysegerät zu spülen bzw. zu desinfizieren und für die nächste Hämodialyse vorzubereiten.

Zusätzlich ist es jedoch auch möglich, das Konzentratansaugrohr in einen Kanister zur Bereitstellung von Konzentratflüssigkeit einzubringen.

Im Folgenden wird die Erfindung anhand zweier Ausführungsbeispiele in Verbindung mit der Zeichnung beschrieben. Dabei zeigt:
- Fig. 1: eine schematische Seitenansicht eines ersten Ausführungsbeispiels des erfindungsgemäßen Druckausgleichsgefäßes und
- Fig. 2: eine schematische Darstellung eines zweiten Ausführungsbeispiels des erfindungsgemäßen Druckausgleichsgefäßes.

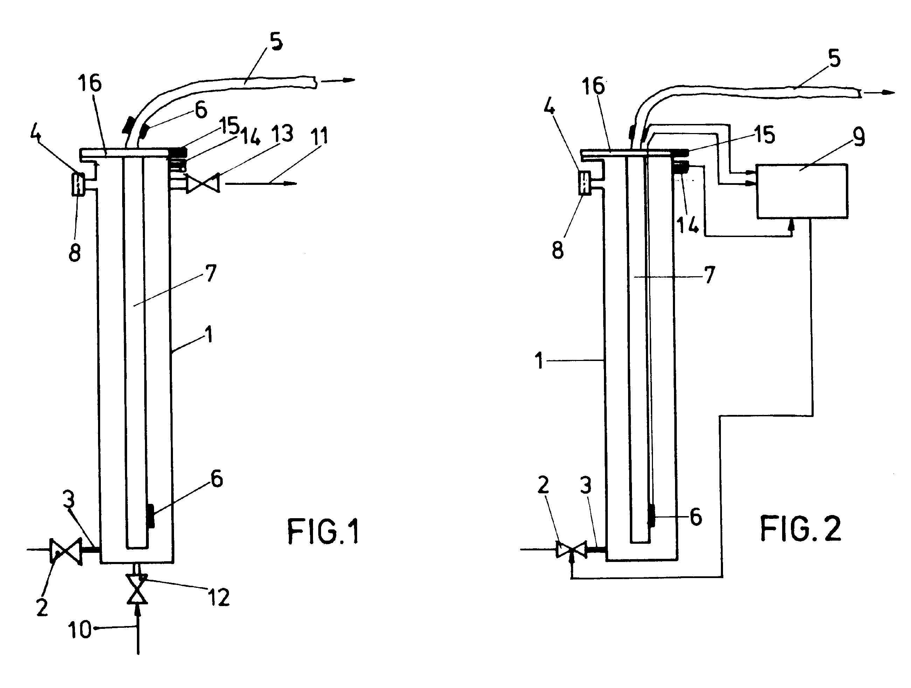

In Fig. 1 ist ein im wesentlichen zylindrisches Gefäß 1 dargestellt, an dessen Bodenbereich eine Einlaßleitung 3 zur Zuführung von Konzentrat vorgesehen ist, welche mittels eines Ventils 2 verschließbar ist. Weiterhin ist im Bodenbereich eine Zuleitung 10 für eine Spülflüssigkeit angeordnet, welche mittels eines Ventils 12 verschließbar ist.

Am oberen Bereich weist das zylindrische Gefäß 1 eine Entlüftungseinrichtung 4 auf, in welcher ein hydrophobes Filter 8 angeordnet ist. Weiterhin ist am oberen Bereich eine Ableitung 11 für Spülflüssigkeit angeschlossen, welche über ein Ventil 13 verschließbar ist.

In das zylindrische Gefäß 1 ist ein Konzentratansaugrohr 7 einsteckbar, welches mit einem Deckel 16 oder einem Flansch verbunden ist und mit einer Leitung 5 zur Abführung der Flüssigkeit in Verbidnung steht, welche üblicherweise an ein Hämodialysegerät angeschlossen ist.

Im Bereich des Konzentratansaugrohrs 7 bzw. der Leitung 5 sind bei dem gezeigten Ausführungsbeispiel Niveausensoren 6 gelagert, welche zu einer Bestimmung der Flüssigkeitsmenge in dem zylindrischen Gefäß 1 und zu einer Regelung auf ein vorgegebenes Flüssigkeitsniveau beitragen. Die Niveausensoren 6 sind beispielsweise in Form von Leitfähigkeitsensoren ausgebildet.

An dem Deckel 16 ist weiterhin ein Magnet 15 befestigt, welcher zur Betätigung eines Magnetschalters 14 dient, mit Hilfe dessen eine exakte Positionierung des Konzentrations-Ansaugrohrs 7 ermittelbar ist.

Während der Dialyse ist das Ventil 2 zeitweise geöffnet, um eine ausreichende Flüssigkeitsmenge in das zylindrische Gefäß 1 einzuführen. Die Flüssigkeit wird durch die Leitung 5 abgeführt, die Ventile 12 und 13 bleiben geschlossen. Während der Spülphase wird das Ventil 2 geschlossen, während die Ventile 12 und 13 geöffnet werden, so daß eine Reinigungsflüssigkeit in das zylindrische Gefäß 1 eingeleitet und sowohl über die Leitung 5 als auch über die Ableitung 11 abgeführt werden kann.

Das in Fig. 2 gezeigte Ausführungsbeipiel entspricht im wesentlichen dem Ausführungsbeispiel gemäß Fig. 1, unterscheidet sich von diesem jedoch darin, daß keine Ableitung 11 bzw. Zuleitung 10 für eine Spülflüssigkeit vorgesehen ist.

In Fig. 2 ist weiterhin eine Steuereinrichtung 9 abgebildet, welche in geeigneter Weise mit den Niveausensoren 6, dem Magnetschalter 14 sowie dem Ventil 2 in Verbindung steht, um die oben beschriebene Funktionsweise des Druckausgleichsgefäßes zu gewährleisten.

Die Erfindung ist nicht auf die gezeigten Ausführungbeispiele beschränkt, vielmehr ergeben sich im Rahmen der Erfindung vielfältige Abwandlungs- und Modifikationsmöglichkeiten.

## Patentansprüche

1. Druckausgleichsgefäß für ein Hämodialysekonzentrat mit einem aufrecht angeordneten Gefäß (1), dessen oberer Bereich mit einer Entlüftungseinrichtung (4) versehen ist und mit einer am oberen Bereich angeordneten Leitung (5) zur Ableitung des Konzentrates zu einem Hämodialysegerät, wobei die Leitung (5) mit einem in dem Gefäß (1) angeordneten Konzentratansaugrohr (7) verbunden ist,
dadurch gekennzeichnet,
daß das Gefäß (1) im wesentlichen zylindrisch ausgebildet ist, daß am unteren Bereich des Gefäßes (1) ein mit einem Ventil (2) versehener Einlaß (3) für Konzentrat angeordnet ist und daß das Konzentratansaugrohr (7) mit zumindest einem Niveausensor (6) versehen ist.

2. Druckausgleichsgefäß nach Anspruch 1, dadurch gekennzeichnet, daß das Konzentratansaugrohr (7) aus dem zylindrischen Gefäß (1) entnehmbar ist und daß das zylindrische Gefäß (1) mittels eines Deckels verschließbar ist.

3. Druckausgleichsgefäß nach Anspruch 1 oder 2, dadurch gekennzeichnet, daß die Entlüftungseinrichtung (4) durch ein hydrophobes Filter (8) verschlossen ist.

4. Druckausgleichsgefäß nach einem der Ansprüche 1 bis 3, dadurch gekennzeichnet, daß das in dem Konzentrateinlaß (3) angeordnete Ventil (2) mittels einer Steuereinrichtung (9) betätigbar ist, welcher Signale von dem Niveausensor (6) zugeführt werden.

5. Druckausgleichsgefäß nach einem der Ansprüche 1 bis 4, dadurch gekennzeichnet, daß der Niveausensor (6) in Form eines Leitfähigkeitssensors ausgebildet ist.

6. Druckausgleichsgefäß nach einem der Ansprüche 1 bis 4, dadurch gekennzeichnet, daß der Niveausensor (6) in Form eines faseroptischen Sensors ausgebildet ist.

7. Druckausgleichsgefäß nach einem der Ansprüche 1 bis 4, dadurch gekennzeichnet, daß der Niveausensor (6) in Form eines Vibrationssensors ausgebildet ist.

8. Druckausgleichsgefäß nach einem der Ansprüche 1 bis 7, dadurch gekennzeichnet, daß das zylindrische Gefäß (1) in Form eines im Hämodialysegerät integrierten Gefäßes ausgebildet ist, mittels dessen das Ansaugrohr (7) während der Desinfektions- und Reinigungsphase desinfizierbar und reinigbar ist.

9. Druckausgleichsgefäß nach Anspruch 8, dadurch gekennzeichnet, daß das zylindrische Gefäß (1) mit einer Spülflüssigkeits-Zuführungsleitung (10) und einer Spülflüssigkeitsableitung (11) versehen ist, in welchen Ventile (12, 13) angeordnet sind, und welche im Dialysierflüssigkeitskreislauf angeschlossen sind.

10. Druckausgleichsgefäß nach Anspruch 9, dadurch gekennzeichnet, daß die Zuleitung (10) und die Ableitung (11) an entgegengesetzten Bereichen des zylindrischen Gefäßes (1) vorgesehen sind.

11. Druckausgleichsgefäß nach einem der Ansprüche 1 bis 10, dadurch gekennzeichnet, daß eine Sensoreinrichtung zur Feststellung einer ordungsgemäßen Anordnung des Konzentrationsansaugrohres (7) an dem zylindrischen Gefäß (1) vorgesehen ist.

12. Druckausgleichsgefäß nach Anspruch 11, dadurch gekennzeichnet, daß die Sensoreinrichtung einen Magnetschalter (14) umfaßt.

## Claims

1. A pressure equalizing vessel for a hemodialysis concentrate, comprising an upright vessel (1) whose upper portion is equipped with a venting means (4), and comprising a conduit (5) arranged on the upper portion thereof for discharging the concentrate to a hemodialysis device, wherein said conduit (5) is connected with a concentrate suction tube (7),
characterized in
that said vessel (1) is substantially cylindrical, that at the lower portion of said vessel (1) a concentrate inlet (3) provided with a valve (2) is arranged and that the concentrate suction tube (7) is provided with at least one level sensor (6).

2. The pressure equalizing vessel according to claim 1, characterized in that said concentrate suction tube (7) can be withdrawn from said cylindrical vessel (1) and that said cylindrical vessel (1) can be closed by means of a cover.

3. The pressure equalizing vessel according to claim 1 or 2, characterized in that said venting means (4) is closed by a hydrophobic filter (8).

4. The pressure equalizing vessel according to any of claims 1 to 3, characterized in that said valve (2) arranged in said concentrate inlet (3) is adapted to be actuated by a control means (9) to which signals from said level sensor (6) are supplied.

5. The pressure equalizing vessel according to any of claims 1 to 4, characterized in that said level sensor (6) is designed in the form of a conductivity sensor.

6. The pressure equalizing vessel according to any of claims 1 to 4, characterized in that said level sensor (6) is designed in the form of a fiber-optical sensor.

7. The pressure equalizing vessel according to any of claims 1 to 4, characterized in that said level sensor (6 ) is designed in the form of a vibration sensor.

8. The pressure equalizing vessel according to any of claims 1 to 7, characterized in that said cylindrical vessel (1) is designed in the form of a vessel which is integrated in said hemodialysis device and by means of which said suction tube (7) can be disinfected and cleansed in the disinfecting and cleansing phase.

9. The pressure equalizing vessel according to claim 8, characterized in that said cylindrical vessel (1) is provided with a flushing liquid supply conduit (10) and a flushing liquid discharge conduit (11) which have arranged therein valves (12, 13) and which are connected in the dialyzing liquid circuit.

10. The pressure equalizing vessel according to claim 9, characterized in that said supply conduit (10) and said discharge conduit (11) are provided on opposite portions of said cylindrical vessel (1).

11. The pressure equalizing vessel according to any of claims 1 to 10, characterized in that a sensing means is provided for sensing the proper arrangement of said concentrate suction tube (7) on said cylindrical vessel (1).

12. The pressure equalizing vessel according to claim 11, characterized in that said sensing means comprises a magnetic switch (14).

## Revendications

1. Réservoir d'égalisation de pression pour un concentré d'hémodialyse comportant un récipient vertical (1), dont la partie supérieure est pourvue d'un dispositif de désaération (4), et une conduite (5) disposée dans la partie supérieure et servant à évacuer le concentré en direction de l'appareil d'hémodialyse, la conduite (5) étant raccordée à un tube (7) d'aspiration du concentré, qui est disposé dans le récipient (1), caractérisé en ce que le récipient (1) est agencé avec une forme essentiellement cylindrique, qu'une entrée (3), pourvue d'une soupape (2), pour le concentré est disposée dans la partie inférieure du récipient (1) et que le tube (7) d'aspiraiton du concentré est équipé d' au moins un détecteur de niveau (6).

2. Réservoir d'égalisation de pression selon la revendication 1, caractérisé en ce que le tube (7) d'aspiration du concentré peut être retiré hors du récipient cylindrique (1) et que le récipient cylindrique (1) peut être fermé au moyen d'un couvercle.

3. Réservoir d'égalisation de pression selon la revendication 1 ou 2, caractérisé en ce que le dispositif de désaération (4) est fermé par un filtre hydrophobe (8).

4. Réservoir d'égalisation de pression selon l'une des revendications 1 à 3, caractérisé en ce que la soupape (2) montée dans l'entrée (3) pour le concentré peut être actionnée au moyen d'un dispositif de commande (9), auquel le détecteur de niveau (6) envoie des signaux.

5. Réservoir d'égalisation de pression selon l'une des revendications 1 à 4, caractérisé en ce que le détecteur de niveau (6) est agencé sous la forme d'un capteur de conductivité.

6. Réservoir d'égalisation de pression selon l'une des revendications 1 à 4, caractérisé en ce que le détecteur de niveau (6) est agencé sous la forme d'un capteur à fibre optique.

7. Réservoir d'égalisation de pression selon l'une des revendications 1 à 4, caractérisé en ce que le détecteur de niveau (6) est agencé sous la forme d'un capteur de vibrations.

8. Réservoir d'égalisation de pression selon l'une des revendications 1 à 7, caractérisé en ce que le récipient cylindrique (1) est agencé sous la forme d'un récipient intégré dans l'appareil d'hémodialyse et au moyen duquel le tube d'aspiration ( 7 ) peut être désinfecté et nettoyé pendant la phase de désinfectation et de nettoyage.

9. Réservoir d'égalisation de pression selon la revendication 8, caractérisé en ce que le récipient cylindrique (1) est équipé d'une conduite (10) d'amenée du liquide de lavage et d'une conduite (11) d'évacuation du liquide de lavage, dans lesquelles sont installées des soupapes (12,13) et qui sont raccordées dans le circuit du liquide de dialyse.

10. Réservoir d'égalisation de pression selon la revendication 9, caractérisé en ce que la conduite d'amenée (10) et la conduite d'évacuation (11) sont prévues sur des parties opposées du récipient cylindrique (1).

11. Réservoir d'égalisation de pression selon l'une des revendications 1 à 10, caractérisé en ce qu'il est prévu un dispositif de détection pour déterminer un montage correct du tube (7) d'aspiration du concentré sur le récipient cylindrique (1).

12. Réservoir d'égalisation de pression selon la revendication 11, caractérisé en ce que le dispositif de détection comprend un interrupteur magnétique (14).
